Europäisches Patentamt

European Patent Office

Office européen des brevets

(19)

(11) Publication number: 0 353 049 A2

# (12) EUROPEAN PATENT APPLICATION

(21) Application number: 89307602.6

(22) Date of filing: 26.07.89

(51) Int. Cl.⁵: **C 12 N 9/04**
C 12 P 9/00, C 12 Q 1/32
//(C12N9/04,C12R1:11)

(30) Priority: 28.07.88 GB 8817967

(43) Date of publication of application:
**31.01.90 Bulletin 90/05**

(84) Designated Contracting States:
AT BE CH DE ES FR GB GR IT LI LU NL SE

(71) Applicant: Genzyme (U.K.) LTD.
37 Hollands Road
Haverhill Suffolk CB9 8PU (GB)

(72) Inventor: Ince, Jane Elizabeth
34 Westfield Blean
Canterbury Kent, CT2 9ER (GB)

Knowles, Christopher John
5 Harbledown Park
Canterbury Kent, CT2 8NR (GB)

(74) Representative: Froud, Clive et al
ELKINGTON AND FIFE Beacon House 113 Kingsway
London WC2B 6PP (GB)

The applicant has filed a statement in accordance with Rule 28 (4) EPC (issue of a sample only to an expert). Accession number(s) of the deposit(s): NCIMB 12470.

(54) NAD-independent alpha- glycerol-3-phosphate dehydrogenases, production and use therof in triglyceride and glycerol determinations.

(57) An enzyme having NAD-independent α-glycerol-3-phosphate dehydrogenase activity characterised in that it is obtained from Bacillus sp. and is not substantially membrane-bound is disclosed, as is the production and use thereof.

EP 0 353 049 A2

**Description**

**NAD - independent α-glycerol-3-phosphate dehydrogenases, production and use thereof in triglyceride and glycerol determinations**

This invention relates to NAD - independent α-glycerol-3-phosphate dehydrogenases and to the production and use thereof in triglyceride and glycerol determinations.

At present, serum triglycerides may be determined clinically using several enzyme-based systems. Two of the principally used schemes which measure triglycerides in biological fluids are illustrated by the following reactions:

$$\text{(a)} \quad \text{triglyceride} \xrightarrow{\text{lipase}} \text{glycerol + fatty acids}$$

$$\text{glycerol + ATP} \xrightarrow{\text{glycerol kinase}} \alpha\text{-glycerol-3-phosphate + ADP}$$

$$\alpha\text{-glycerol-3-phosphate} + O_2 \xrightarrow{\alpha\text{-glycerol-3-phosphate oxidase}} \text{dihydroxyacetone phosphate} + H_2O_2$$

$$\text{H}_2\text{O}_2 + \text{phenol} + 4\text{-aminoantipyrine} \xrightarrow{\text{peroxidase}} \text{quinone imine dye}$$

$$\text{(b)} \quad \text{triglyceride} \xrightarrow{\text{lipase}} \text{glycerol} + \text{fatty acids}$$

$$\text{glycerol} + \text{ATP} \xrightarrow{\text{glycerol kinase}} \alpha\text{-glycerol-3-phosphate} + \text{ADP}$$

$$\alpha\text{-glycerol-3-phosphate} + \text{NAD}^+ \xrightarrow{\substack{\text{NAD}^+\text{-dependent} \\ \alpha\text{-glycerol-} \\ \text{3-phosphate} \\ \text{dehydrogenase}}} \text{dihydroxyacetone phosphate} + \text{NADH}$$

$$\text{NADH} + \text{colourless INT} \xrightarrow{\text{diaphorase}} \text{NAD}^+ + \text{red reduced INT}$$

Generally, the present invention relates to enzymes having $\text{NAD}^+$ and $\text{NADP}^+$ independent $\alpha$-glycerol-3-phosphate dehydrogenase activity. In one embodiment the present invention provides an enzyme having NAD-independent $\alpha$-glycerol-3-phosphate dehydrogenase activity characterised in that it is obtainable from Bacillus sp. and is not substantially membrane-bound. Such enzymes catalyse the transfer of electrons from $\alpha$-glycerol-3-phosphate directly to acceptors, such as INT (2-(4-iodophenyl)-3-(4-nitrophenyl)-5-phenyltetrazolium chloride; p-iodonitrotetrazolium violet), DCPIP (2,6-dichlorobenzenone; 2,6-dichloroindophenol), MTT (3-(4,5-dimethyl thiazole-2)-2, 5-diphenyl tetrazolium; thiazolyl blue), PMS (N-methyldibenzopyrazine methyl sulphate; phenazine methosulphate) and NBT (2,2′-di-p-nitrophenyl-5,5′-diphenyl-3,3′-(3.3′-dimethoxy-4,4′-diphenylene) ditetrazolium; nitro blue tetrazolium), and alleviate the need for the reaction catalysed by diaphorase enzyme in scheme (b) illustrated above. While the present invention relates generally to the use of such enzymes to catalyse the dehydrogenation of $\alpha$-glycerol-3-phosphate, they are particularly useful in triglyceride/glycerol/$\alpha$-glycerol-3-phosphate determinations. In one embodiment, the present invention relates to a method of triglyceride/glycerol/$\alpha$-glycerol-3-phosphate determination characterised in that it comprises producing a sample containing $\alpha$-glycerol-3-phosphate and using such an enzyme to catalyse a colourimetric reaction thereof to give the desired indication, the glycerol-3-phosphate optionally having been produced from glycerol previously and the glycerol optionally having been produced from triglyceride previously. In another embodiment, the present invention relates to a test kit for triglyceride/glycerol/$\alpha$-glycerol-3-phosphate determination characterised in that it comprises means for providing a sample containing $\alpha$-glycerol-3-phosphate, such an enzyme and means to effect a characteristic reaction thereof to give the desired indication, also optionally means for providing an α-glycerol-3-phosphate - containing sample from a glycerol -containing sample and optionally means for providing a glycerol - containing sample from a triglyceride - containing sample.

According to the present invention, in particular triglycerides and glycerol may be determined by methods based on the following illustrative scheme:

$$\text{lipase}$$

$$\text{(c)} \quad \text{triglyceride} \longrightarrow \text{glycerol + fatty acids}$$

$$\text{glycerol}$$

$$\text{kinase}$$

$$\text{glycerol + ATP} \longrightarrow \alpha\text{-glycerol-3-phosphate + ADP}$$

$$\alpha\text{-glycerol-3-phosphate + colourless INT}$$

$$\text{NAD}^+\text{-independent}$$

$$\alpha\text{-glycerol-3-phosphate}$$

$$\text{dehydrogenase}$$

$$\text{dihydroxyacetone phosphate + red reduced INT}$$

The present invention may be applied to the determination of triglycerides and glycerol from various sources, for example the estimation of triglycerides in foodstuffs and in the blood.

A preferred enzyme according to the present invention may be obtained from Bacillus sp., more particually B. megaterium. Such an enzyme has been reported in B. subtilis (Oh, Y. K., et al, J. Bact., 113, 1034-45, (1973).). In this scientific journal paper, NAD-independentα-glycerol-3-phosphate dehydrogenase activity was measured by the reduction of MTT in the presence of PMS as a mediator. Other bacterial sources of such enzymes have been reviewed by Lin, E.C.C., Ann. Rev. Microbiol., 30, 535, (1976). These include Escherichia coli. Klebsiella aerogenes and Rhodopseudomonas. Cellulomonas gelida has also been mentioned as a source (see Nishise, H., et al, Agric. Biol. Chem., 49. 629-636, (1985)). The enzyme from E. coli has been purified. It is membrane bound and extraction requires solubilisation with detergent plus a high salt concentration (see Weiner, J.H., and Heppel, L.A., Biochem. Biophys. Res. Com., 47, 1360-1365, (1972).).

Enzymes according to the present invention may be obtained from various sources by generally conventional means well within the competence of those skilled in the art. In one embodiment, the present invention relates to a process for the production of such an enzyme characterised in that it comprises culturing an appropriate bacterium, harvesting and disrupting the cells, separating a cell-free extract and at least partially purifying the desired enzyme.

Such an enzyme may be obtained from Bacillus, sp. in particular B. megaterium, by culturing the organism in the following exemplary manner: A nutrient agar slant may be used to inoculate 100ml of medium containing, for example, glycerol (4-l0gl⁻¹), yeast extract (4gl⁻¹), either mycological peptone (2- 4gl⁻¹) or bacteriological peptone (2-10gl⁻¹), $K_2HPO_4$ (5gl⁻¹) , $NaH_2PO_4.2H_2O$ (1gl⁻¹), $CaCl_2$ (0.1mM), $MgSO_4.7H_2O$ (1.0mM) and trace quantities of cobalt, manganese, borate, copper, zinc and iron (II). The medium may be sterilised by autoclaving or filtration and has an initial pH of 7.6. The culture may be grown by shaking in a rotary shaker (150-300rpm) at 30°C for 8-12 hours. This seed may be used to inoculate large shake flasks or fermenters at an inoculum density of from 1 to 10%. A culture in a larger shake flask may be grown on a rotary shaker (150-300rpm) at 30°C for 8-16 hrs. A culture in a fermenter may be grown at 30°C, agitated at 500-1000rpm with the pH controlled 7.0 - 7.6 for 8-12 hours.

The culture may be harvested by centrifugation and may be resuspended in a buffer, for example pH 7.0 - 7.6 containing detergent, for example, "Triton", "Brij" or "Tween". The desired enzyme may be released into solution from the cells by generally conventional means and the resultant cell debris removed by centrifugation. As will be appreciated by those skilled in the art, the specific activity of the enzyme released may vary with the culture conditions and generally may vary from 0.03 to 0.48 U/mg protein. The purification of the desired enzyme may be carried out by generally conventional means, such as gel filtration or ion exchange chromatography well within the competence of those skilled in the art.

The preferred enzyme which may be obtained from Bacillus, sp., specifically B. megaterium, has several advantages over other known NAD⁺ independent enzymes particularly for use in triglyceride and glycerol determinations. For example, it has a comparatively high affinity (low Km) for the substrate L-α-glycerol-3-phosphate (Km = 0.178 mM with INT as the electron acceptor). This compares with Km values of 0.4mM for the enzyme from E. coli (Weiner and Heppel, loc cit) and 2.3mM for the enzyme from Rhodopseudomonas capsulata (see Lucking, B., et al, J. Bact., 115, 897-903, (1973)). It is not necessary to include mediators, such as PMS or menadione, in assays using the preferred enzyme from Bacillus, sp. bacterium. This enzyme is extracted more easily than from other sources. Although some detergent is used, a high salt concentration is not necessary. Using DCPIP as the electron acceptor, an activity of $62 \times 10^{-3}$ mmol per min per litre of culture is obtained. A large proportion of the enzyme activity (60%) was found to be soluble in cell-free extracts when they were fractionated by high speed centrifugation. One important feature of the preferred enzyme is that it is, partially at least soluble. Known examples of such enzymes are membrane-bound. This is particularly advantageous in the recovery of the preferred enzyme according to the present invention.

As mentioned above, numerous different electron acceptors may be used in conjunction with enzymes according to the present invention. In addition to such dye-based colourimetric assays the present enzymes may be used in other assay systems, such as electro-chemical sensors.

One aspect of the present invention relates to the improved enzyme and another to the production thereof from suitable sources. Such enzymes are particularly useful in triglyceride and glycerol determinations and may be provided as components of kits in accordance with the aspects of the present invention.

By way of further illustration, the preferred enzyme obtained from B. megaterium may be more particularly characterised as follows:

The molecular weight (determined by gel filtration) is approx. 450,000. The enzyme contains sub-units of molecular weight 71,000 and 19,000 (determined by SDS-polyacrylamide gel electrophoresis). The pH optimum was greater than pH 8.5 using Tris buffer. Approximately 50% of the maximal activity was seen at pH 7.5. The enzyme has a Michaelis-Menten constant (Km) in respect of its substrate, L-α-glycerol-3-phosphate, of 0.178 mM. The activity is stable to dialysis. Using the oxygen electrode, it was found that the rate of oxygen uptake in the presence of L-α-glycerol-3-phosphate was only 10% of the rate of INT reduction for the same amount of cell-free extract. Therefore, oxygen is not the initial acceptor for the enzyme. (The conversion of L-α-glycerol-3-phosphate to reduced INT was stoichiometric.) The enzyme appears stable to freezing. When diluted to 0.5 mg protein/ml in phosphate buffer, pH 7.5, cell-free extract retains 70% of its enzyme activity when stored for 5 days at 4°C.

The enzyme activity may be inhibited by a number of compounds, for example 50% inhibition may be brought about by the following: cholate (0.03%), ammonium sulphate (1%) and deoxycholate (<0.03%). The enzyme activity is completely inhibited by $Zn^{2+}$ at a concentration of 11mM.

The recovery of the preferred enzyme may be more particularly illustrated by the following:

A culture of Bacillus sp. was obtained as a contaminant while growing another bacterium. A nutrient agar slant was used to inoculate 100ml of medium containing glycerol (8g l⁻¹), yeast extract (4g l⁻¹), mycological peptone (2g l⁻¹)), $K_2HPO_4$ (5g l⁻¹), $NaH_2PO_4.2H_2O$ (1g l⁻¹), $CaCl_2$ (0.1mM), $MgSO_4.7H_2O$ (1.0mM) and trace quantities of cobalt, manganese, borate, copper, zinc and iron (II). The medium was sterilised by autoclaving and had an initial pH of 7.6. The cultures were allowed to grow with shaking in a rotary shaker (200 rpm) at 30°C for 12h. 10ml of this seed culture was used to inoculate 500ml of the same medium and the culture allowed to grow with shaking in a rotary shaker (200 rpm) at 30°C for 12h. The microorgansims were harvested by centrifugation at 18,000 x g and resuspended in 0.05M $Na_2HPO_4/NaH_2PO_4$ buffer, pH 7.5, containing 0.5% (v/v) "Triton-x-100" at 4°C. The cell suspension was subjected to disruption by sonication using an MSE (Model 150W) sonicator with 7 x 20s bursts of sonication. The cell debris was removed from the homogenate by centrifugation at 18,000 x g. The supernatant was decanted and is termed cell-free extract. The specific activity of α-glycerol-3-phosphate dehydrogenase in the cell-free extract was 0.03 U/mg protein.

A nutrient agar slant of Bacillus sp. was used to inoculate 100ml of medium containing glycerol (8gl⁻¹) yeast extract (4gl⁻¹), mycological peptone (2gl⁻¹), $K_2HPO_4$ (5gl⁻¹), $NaH_2PO_4.2H_2O$ (1gl⁻¹), $CaCl_2$ (0.1mM), $MgSO_4.7H_2O$ (1.0mM) and trace quantities of cobalt, manganese, borate, copper, zinc and iron (II). The medium was sterilised by a autoclaving and had an initial pH of 7.6. The cultures were grown with shaking in a rotary shaker (200 rpm) at 30°C for 12h. 100ml of this seed culture was used to inoculate a fermenter containing 1500ml of the following, glycerol (6gl⁻¹), yeast extract (4gl⁻¹), mycological peptone (2gl⁻¹), $K_2HPO_4$ (5gl⁻¹) $NaH2PO_4.2H_2O$ (1gl⁻¹), $CaCl_2$ (0.1mM), $MgSO_4.7H_2O$ (1.0mM) and trace quantities of cobalt manganese, borate, copper, zinc and iron (II). 1ml of PPG was added for foam control. The medium was sterilised by autoclaving and had a initial pH of 7.6. The cultures were grown for 8 hours in the fermenters under the following conditions: agitation 500rpm, air 0.5l/ min, temperature 30°C, pH controlled to 7.6 with 3M NaOH. The microorganisms were harvested by centrifugation at 18000 x g and resuspended in 6mM dimethyl glutarate buffer, pH 7.5, to a cell concentration of 10%. The cell suspension was subjected to disruption by sonication for up to 13 minutes at 75% power input. The cell debris was removed from the homogenate by centrifugation at 18000 x g. The supernatant was decanted and termed the cell-free extract. The specific activity of α-glycerol-3-phosphate dehydrogenase in the cell-free extract was 0.186 U/mg protein.

Aliquots of the resulting cell-free extract and other samples containing partially purified α-glycerol-3-phosphate dehydrogenase were used in the following assay test system:

| | |
|---|---|
| DL-α-glycerol-3-phosphate | 2.0mM |
| INT | 0.5mM |
| Dimethylglutarate buffer, pH 7.5 | 60mM |

The rate of change of absorbance was measured at 505nm, 37°C in a spectrophotometer. According to such test, the specific activity of the purified enzyme (from gel filtration chromatography) was 1.0 unit/mg protein; a unit is number of $\mu$moles of INT reduced per min. With a view to partial purification of enzyme, a portion of cell-free extract was spun at high speed for 90 min (100,000 x g). The high speed supernatant was applied to a chromatography column (1.6 x 150 cm) containing "Sephadex G-200" gel filtration resin. The proteins were eluted with 50mM $Na_2HPO_4$ buffer, pH 7.5, the flow rate was 7ml $h^{-1}$. The eluate was collected in 1.5ml fractions and was assayed by the previously described test system to determine the point of elution of α-glycerol-3-phosphate dehydrogenase activity. The enzyme was found to elute shortly after the void volume of the column with a molecular weight of 450,000. The pooled active fractions contained a specific activity of 1.0 U/mg protein.

α-glycerol-3-phosphate dehydrogenase was also purified from the cell-free extract using anion exchange chromatography. Cell-free extract was applied to a column packed with DEAE Sepharose resin equilibrated with 50mM Tris buffer, pH 7.5. The activity became bound to the resin and was eluted using a gradient of increasing NaCl concentration (0 to 1.0M) in 50mM Tris buffer, pH 7.5. The eluate was collected in 4ml fractions and was assayed by the previously described test system. The enzyme eluted between 0.3 and 0.8M NaCl. The pooled active fractions contained a specific activity of 0.36 U/mg protein.

Addition of 15% polyethylene glycol to cell-free extracts caused precipitation of α-glycerol-3-phosphate dehydrogenase which could be recovered by redissolving the pellet. The process resulted in a doubling of the specific activity to 0.05 U/mg protein. The precipitation of the enzyme with PEG may be improved by the addition of $Zn^{2+}$ to a concentration of 33mM. $Zn^{2+}$ alone may be used to precipitate the enzyme when added to a concentration of 33mM. The inhibition of the enzyme activity by $Zn^{2+}$ may be overcome by resuspending the enzyme precipitate in 30mM dimethyl glutarate buffer, pH 7.5, containing 50mM EDTA. This process resulted in a 1.6-fold improvement in specific activity with a 91% recovery of enzyme activity.

α-glycerol-3-phosphate dehydrogenase was purified from the resuspended $Zn^{2+}$ precipitate using anion exchange chromatography. The redissolved $Zn^{2+}$ precipitate was applied to a column packed with QA cellulose equilibrated with 50mM Tris buffer, pH 7.5. The activity was bound to the cellulose and was eluted using a gradient of increasing NaCl concentration (0 to 1.0m) in 50mM Tris buffer, pH 7.5. The enzyme eluted between 0.7 and 1.0M NaC1. The pooled active fractions contained a specific activity of 0.96 U/mg protein.

A non-denaturing polyacrylamide electrophoresis gel (7.5%) was run with samples of cell-free extract and protein recovered from batch ion exchange using DE 52 resin. After running, the gel was cut in half and stained for protein or with an activity stain containing DL-α-glycerol-3-phosphate and INT. An intense pink band in the activity stain gel corresponded to one of the two major bands seen in the protein stain. SDS polyacrylamide gel electrophoresis of the pooled active fractions from the gel filtration chromatography demonstrated that the enzyme was composed of sub-units of molecular weight 71,000 and possibly a lighter type of sub-unit, of molecular weight 19,000.

(The full designation of the above-mentioned B. megaterium is Bacillus megaterium JEI. It was deposited at the National Collection of Industrial and Marine Bacteria on 16 February 1987 and has been accorded accession number NCIMB 12470.)

## Claims

1. An enzyme having NAD-independent α-glycerol-3-phosphate dehydrogenase activity characterised in that it is obtainable from Bacillus sp. and is not substantially membrane-bound.

2. An enzyme as claimed in claim 1 wherein it is obtained from B. megaterium JEI (NCIMB 12470).

3. An enzyme as claimed in claim 2 wherein it has a Km of about 0.178 mM.

4. A process for the production of an enzyme as claimed in any of claims 1 to 3 characterised in that it comprises culturing an appropriate bacterium, harvesting and disrupting the cells, separating a cell-free extract and at least partially purifying desired enzyme.

5. The use of an enzyme as claimed in any of claims 1 to 3 to catalyse the dehydrogenation of α-glycerol-3-phosphate.

6. A method of triglyceride/glycerol/α-glycerol-3-phosphate determination characterised in that it comprises producing a sample containing α-glycerol-3-phosphate and using an enzyme as claimed in any of claims 1 to 3 to catalyse a colourimetric reaction thereof to give the desired indication, the glycerol-3-phosphate optionally having been produced from glycerol previously and the glycerol optionally having been produced from triglyceride previously.

7. A test kit for triglyceride/glycerol/α-glycerol-3-phosphate determination characterised in that it comprises means for providing a sample containing α-glycerol-3-phosphate, an enzyme as claimed in any of claims 1 to 3 and means to effect a characteristic reaction thereof to give the desired indication, also optionally means for providing an α-glycerol-3-phosphate -containing sample from a glycerol -containing sample and optionally means for providing a glycerol - containing sample from a triglyceride - containing sample.